Europäisches Patentamt

European Patent Office

Office européen des brevets

(19)

(11) Numéro de publication: **0 292 404**
**A1**

(12) **DEMANDE DE BREVET EUROPEEN**

(21) Numéro de dépôt: 88401236.0

(22) Date de dépôt: 20.05.88

(51) Int. Cl.⁴: **G 01 N 33/569**
C 12 N 15/00, A 61 K 39/21

(30) Priorité: 22.05.87 FR 8707263

(43) Date de publication de la demande:
23.11.88 Bulletin 88/47

(84) Etats contractants désignés:
AT BE CH DE ES FR GB GR IT LI LU NL SE

(71) Demandeur: TRANSGENE S.A.
16, rue Henri Régnault
F-92400 Courbevoie (FR)

INSTITUT PASTEUR DE LILLE
1, rue du Professeur Calmette BP 245
F-59019 Lille Cédex (FR)

INSTITUT PASTEUR
25, rue du Docteur Roux
F-75015 Paris (FR)

(72) Inventeur: Capron, André
58, rue du Capitaine Jasmin
F-59113 Phalempin (FR)

Khalife, Jamal
1, allée du Général Koening
F-59130 Lambersart (FR)

Capron, Monique
58, rue du Capitaine Jasmin
F-59113 Phalempin (FR)

Tartar, André
rue du Moulin
F-62490 Vitry en Artois (FR)

Montagnier, Luc
21, rue de Malabry
F-92350 Le Plessis-Robinson (FR)

Guy, Bruno
18, rue Sainte-Madeleine
F-67000 Strasbourg (FR)

Lecocq, Jean-Pierre
6, rue du Champ du Feu
F-67460 Reichstett (FR)

(74) Mandataire: Warcoin, Jacques et al
Cabinet Régimbeau 26, avenue Kléber
F-75116 Paris (FR)

(54) **Test de diagnostic du SIDA basé sur la mise en évidence d'une diversité de la réponse isotypique, vaccins et sérothérapies.**

(57) La présente invention concerne la mise au point d'un nouveau test de diagnostic du SIDA par l'immunocapture d'anticorps de différents isotypes et par l'immunodétection de l'antigène F, de systèmes de vaccins et de sérothérapies.

L'invention permet un affinement du diagnostic et pronostic de l'évolution de la maladie ainsi que la conception de vaccins et de sérothérapies adaptés aux caractéristiques particulières de la maladie.

EP 0 292 404 A1

**Description**

## TEST DE DIAGNOSTC DU SIDA BASE SUR LA MISE EN EVIDENCE D'UNE DIVERSITE DE LA REPONSE ISOTYPIQUE, VACCINS ET SEROTHERAPIES.

La présente invention concerne un nouveau test de diagnostic du SIDA ; ce test permet de révéler toutes les classes d'anticorps qui apparaissent après une contamination par le virus du SIDA et ainsi de détecter des serums qui apparaissent, à tort, comme séronégatifs dans les "kits" de détection d'anticorps actuellement disponibles ; ce test permet aussi d'affiner le pronostic de l'évolution de la maladie.

Plus particulièrement, la présente invention concerne :
- d'une part, l'utilisation à des fin diagnostiques de l'immunonocapture de certaines classes d'anticorps, appelées isotypes, qui ne sont pas reconnues par les tests actuels, et
- d'autre part, l'immunodétection d'anticorps dressés contre la protéine F du virus LAV (ou HIV), protéine qui ne fait pas partie des "kits" actuels et qui apparaît comme un marqueur important de l'infection par le virus HIV.

Les hypothèses de travail qui ont mené à la présente invention sont basées sur des observations et analyses des mécanismes immunitaires qui interviennent dans des maladies parasitaires, en particulier la Schistosomose et la Toxoplasmose.

Les travaux sur l'immunogénicité et le pouvoir protecteur de différentes protéines des schistosomes ont montré que des protéines excrétées-sécrétées, et transitoirement exprimées au niveau de la membrane, jouent un rôle dans l'élaboration de la réponse immune protectrice.

Par contre, les anticorps dressés contre les antigènes de membrane apparaissent plus comme des marqueurs d'infection que comme des effecteurs dans les mécanismes de défense contre l'infection. Il semble en effet normal que lors d'une infection chronique, quand le parasite survit dans l'individu en présence d'une immunité concomitante, les antigènes de surface ne soient pas les cibles de l'immunité puisqu'ils sont essentiels à la survie du parasite. De plus, des travaux récents ont montré que ces antigènes de membrane induisaient des anticorps bloquants, capables d'inhiber la réponse cytotoxiquedépendante des anticorps.

Dans ces modèles d'infection parasitaire, l'analyse des mécanismes effecteurs et immunorégulateurs souligne l'importance et le rôle particulier de certaines classes d'anticorps et fournit une illustration du phénomène de sélection et de restriction isotypique. (On trouve une mise au point récente sur l'interprétation au niveau moléculaire des phénomènes de restriction isotypique dans la revue de Cebra et al. 1984.)

Les travaux réalisés sur la Schistosomose (revue dans Capron et Capron, 1986) indiquent clairement l'existence d'une sélection isotypique dans la reconnaissance de certains antigènes et l'induction par un même antigène, voire un même épitope, d'isotypes effecteurs et d'isotypes bloquants (dans les modèles animaux - Grzych et al. 1984 - et chez l'homme - Khalife et al. 1986).

L'ensemble de ces observations a permis de dégager le concept qu'une réponse anticorps telle qu'elle est mesurée globalement, dans les tests habituels, n'est fonctionnellement que la résultante d'activités effectrices et bloquantes liées à certains isotypes et que l'efficacité d'une réponse immune est aussi dépendante des facteurs négatifs de régulation que des marqueurs positifs de protection (Capron et al. 1983).

Les concepts originaux qui se dégagent de ces travaux peuvent être appliqués à d'autres infections chroniques et en particulier à des infections virales à évolution lente et progressive comme celle du virus du SIDA (HIV).

Par certaines de ses caractéristiques (ancienneté d'évolution, variation antigénique, latence, chronicité, multiplication intracellulaire, mécanisme d'échappement à la réponse immunitiare de l'hôte), le virus HIV semble répondre à certains critères de mode d'infection des modèles parasitaires précédemment étudiés. Bien que de nombreuses études sérologiques aient été entreprises sur l'infection par le HIV et que l'élévation de certaines classes ou sous-classes d'anticorps ait été rapportée, aucun travail d'ensemble n'a encore été entrepris dans le but de déterminer, pour chacune des protéines virales, l'existence d'une réponse isotypique sélective ou restreinte. Cette étude est particulièrement importante parce que le test de diagnostic du SIDA couramment utilisé et qui détecte la présence d'anticorps dans les serums humains ne révèle pas tous les isotypes. En effet le test comprend, comme révélateur de séropositivité, des anti-IgG totales. Ces anti-IgG ne détectent que faiblement les IgG 4 et ne détectent pas les anticorps de classe IgM, IgA et IgE.

Des individus positifs seulement pour les anticorps de ces isotypes apparaissent comme négatifs dans le test. Or certains stades de l'évolution de la maladie, en particulier les stades précoces, peuvent donner une réponse isotypique différente des stades ultérieurs et donc échapper à la détection.

Il faut aussi noter que la mise en évidence d'anticorps d'isotype IgG4, IgA et IgM (qui correspondent généralement à des anticorps bloquants) peut expliquer les faibles taux d'anticorps séroneutralisants détectés dans les serums de malades.

La présente invention repose, d'une part, sur l'immunocapture, à l'aide d'anti-anticorps spécifiques d'isotypes, des IgG1, IgG3 et IgG4, des IgM, des IgA et des IgE dressés contre les diverses protéines exprimées par le virus HIV et en particulier les protéines codées par le gène gag et le gène F, ou encore des peptides dérivés de la protéine F, en particulier les peptides correspondant aux acides aminés 45 à 69 et 98 à 112. Ces peptides peuvent être préparés par synthèse in vitro.

C'est pourquoi l'invention a également pour objet deux nouveaux peptides, de séquence :

45 à 69 : SSNTAATNAACAWLEAQEEEEVGFP

98 à 112 : EGLIHSQRRQDILDL

La présente invention repose, d'autre part, sur la mise en évidence d'une réponse immune contre la protéine F du virus HIV, protéine qui ne fait pas partie des "kits" actuels ou contre les peptides dérivés de la protéine F tels qu'ils ont été définis ci-dessus. Même si le rôle de la protéine F dans le mécanisme infectieux n'est pas encore établi de manière définitive, la mise en évidence d'une réponse anti-F permet d'affiner le diagnostic et de donner des indications sur l'état d'avancement de la maladie.

Enfin, l'invention repose sur l'utilisation de nouvelles protéines à titre d'agents vaccinants.

Plus particulièrement, la présente invention concerne un procédé de diagnostic in vitro du SIDA chez un patient, par mise en évidence des anticorps dressés contre certaines protéines spécifiques du virus HIV dans un prélèvement biologique dudit patient, selon un processus d'immunodétection indirect dans lequel :

a) on fixe la ou lesdites protéines du virus HIV, séparées, sur un support,

b) on met en présence les protéines fixées avec le prélèvement biologique dudit patient pouvant contenir lesdits anticorps, et

c) après l'immunocapture des anticorps, on détecte la présence des anticorps fixés à l'aide d'anti-anticorps marqués, procédé caractérisé en ce que parmi les anti-anticorps marqués assurant la détection, l'un au moins est spécifique d'un isotype.

Le processus d'immunodétection indirect est connu de l'homme de métier sans qu'il soit nécessaire de le décrire en détail. Des informations complémentaires pourront être trouvées dans les exemples.

Le prélèvement biologique testé sera, en général, un sérum.

Le support peut être quelconque, mais sera la plupart du temps constitué par des bandelettes de nitrocellulose.

De même, pour les méthodes de détection des anti-anticorps, il s'agit de techniques connues. Les anti-anticorps sont marqués par une enzyme, la détection étant effectuée par addition du substrat de l'enzyme, ou bien les anti-anticorps sont marqués par un isotope radioactif. Les conditions de mise en oeuvre de ces différentes étapes sont connues, certaines sont décrites en détail dans les exemples.

De préférence, afin de permettre une analyse fine permettant un diagnostic fiable, on utilisera au moins deux anti-anticorps.

De préférence, on utilisera un ensemble d'anti-anticorps couvrant tous les isotypes.

Parmi les isotypes intéressants qui ne sont pas détectés par les "kits" actuels qui détectent les IgG totaux, il faut citer les IgM, les IgA et les IgE.

De même, il est préférable de pouvoir détecter séparément les différents isotypes afin d'affiner le pronostic quant à l'évolution de la maladie.

Parmi les protéines fixées peuvent figurer une ou plusieurs des protéines correspondant au gène GAG, POL, ENV et les peptides dérivés de ces protéines et/ou la protéine F ou les peptides dérivés de la protéine F.

Bien entendu, ces différentes protéines sont séparées par électrophorèse sur gel de polyacrylamide (SDS-PAGE) puis tranférées sur membrane de nitrocellulose comme cela est usuel, mais il est possible de prévoir d'autres traitements de séparation.

Ces protéines peuvent, dans certain cas, être obtenues par recombinaison génétique, par exemple la protéine env, la protéine F et la protéine p25 ; c'est ce type de protéine qui sera plus particulièrement utilisé dans le procédé de l'invention.

Dans les procédés selon l'invention, outre les trousses comportant au moins une ou plusieurs protéines du virus HIV préalablement isolées choisies parmi les protéines correspondant au gène :

- GAG (p55, p40, p25, p18 et p13)
- POL
- ENV (gp160, gp120 et gp40)
- protéine F,

et au moins un anti-anticorps spécifique d'un isotype choisi parmi IgG1, IgG3, IgG4, IgA, IgM, IgE, les trousses suivantes sont plus particulièrement intéressantes :

- les trousses de diagnostic caractérisées en ce qu'elles comportent au moins la protéine F et la protéine env et au moins un anti-anticorps spécifique des isotypes IgG1, IgG4, IgA IgM ou IgE, et

- les trousses de diagnostic caractérisées en ce qu'elles comportent au moins la protéine env, la protéine p25 ou la protéine correspondant au gène GAG et au moins un anti- anticorps spécifique de l'isotype IgA, IgE, IgG1, IgG3, IgG4 et/ou IgM.

Bien entendu, il est toujours possible d'adjoindre d'autres isotypes.

De même, des trousses contenant la protéine F ou des peptides dérivés de celle-ci parmi les antigènes détectables (ELISA ou Western Blot) peuvent permettre d'établir un diagnostic précoce et d'éliminer certains faux négatifs. Dans ce cas, des anti-anticorps spécifiques des différents isotypes ou des anti-IgG totaux peuvent être utilisés.

Par ailleurs, la mise en évidence de ces isotypes permet d'envisager des vaccins contre le SIDA présentant des caractères novateurs par rapport aux conceptions actuelles. En effet, comme cela a été indiqué précédemment, certains des anticorps induits sont de type effecteur mais d'autres sont de type bloquant. Seuls les antigènes produisant une réponse de type effecteur peuvent permettre une immunisation.

C'est pourquoi la présente invention concerne des vaccins destiné au traitement et à la prévention des affections liées à la présence d'un virus HIV chez un patient, caractérisés en ce qu'on utilise comme agent vaccinant une protéine antigénique du virus HIV modifiée pour induire des anticorps d'isotypes effecteurs et non bloquants.

Parmi ces protéines antigéniques du virus HIV on choisira, de préférence, les protéines modifiées capables d'induire des anticorps de type effecteur.

Les protéines à modifier seront comme précédemment celles correspondant au gène GAG, POL, ENV, la protéine F ou Q ou les peptides dérivés de ces protéines.

Enfin, on peut envisager la mise en oeuvre de sérothérapies nouvelles. En effet, un transfert passif d'anticorps effecteurs produits chez l'homme, sans qu'il ait été nécessairement exposé au HIV, ou dans des animaux ou des cellules en culture, peut être envisagé dans le traitement des individus séropositifs asymptomatiques ou présentant des signes cliniques. On sait qu'une telle thérapie a des effets bénéfiques dans le traitement d'infections parasitaires (Schistosomiase expérimentale) ou le traitement des tumeurs (cancer colorectal) tant chez l'homme que chez l'animal.

On peut envisager aussi la préparation et l'utilisation d'anticorps monoclonaux.

La présente invention concerne donc l'utilisation d'anticorps effecteurs, notamment d'isotypes particuliers à effet séroneutralisant et/ou cytotoxique, contre les protéines du virus HIV dans le traitement du SIDA. Les anticorps effecteurs peuvent être obtenus par immunisation au moyen de protéines modifiées comme décrit précédemment, ou par séparation des isotypes naturels et élimination des isotypes bloquants.

D'autre part, dans le cas où des anticorps à activité bloquante ont été détectés chez des individus séropositifs, il pourra être envisagé l'injection d'anticorps dirigés contre ces anticorps bloquants. Ces anticorps pourront être d'origine animale.

Les figures suivantes illustrent les exemples :

Figure 1 : Electrophorèse sur gel d'acrylamide 15 % - SDS des extraits de bactéries transformées par les plasmides pTG959 (1) témoin et pTG1166 (2) portant le gène F.

Figure 2 : Immunocapture et détection spécifique des anticorps de classe IgA dans le serum de 27 patients. La bandelette témoin révélée avec des IgG (témoin du test LAV-BLOT) indique la position et la nature des différentes protéines du HIV.

Figure 3 : Immunocapture et détection spécifique des anticorps de classe IgM dans le serum de 22 patients. La bandelette témoin révélée avec des IgG (témoin du test LAV-BLOT) indique la position et la nature des différentes protéines du HIV.

Figure 4 : Immunocapture et détection spécifique des anticorps de classe IgE dans le sérum de 4 patients. La bandelette témoin révélée avec des IgG (témoin du test LAV-BLOT) indique la position et la nature des différentes protéines du HIV.

Figure 5 : Immunocapture et détection spécifique des anticorps d'isotype IgG1 dans le serum de 25 patients. La bandelette témoin révélée avec des IgG (témoin du test LAV-BLOT) indique la position et la nature des différentes protéines du HIV.

Figure 6 : Immunocapture et détection spécifique des anticorps d'isotype IgG3 dans le serum de 25 patients. La bandelette témoin révélée avec des IgG (témoin du test LAV-BLOT) indique la position et la nature des différentes protéines du HIV.

Figure 7 : Immunocapture et détection spécifique des anticorps d'isotype IgG4 dans le serum de 25 patients. La bandelette témoin révélée avec des IgG (témoin du test LAV-BLOT) indique la position et la nature des différentes protéines du HIV.

Figure 8 : Immunocapture et détection spécifique des isotypes IgG1, IgG3, IgG4, IgA, IgM et IgE dans le serum de quelques patients particuliers (les réponses des patients N°6 et N°20 sont analysées dans l'exemple 3).

Figure 9 : Immunocapture et détection des anticorps dressés contre la protéine F du virus HIV dans le sérum de 43 patients. La protéine F fixée sur la nitrocellulose a été produite par manipulation génétique dans E. coli. Les anticorps sont révélés avec des anti-IgG totales.

Figure 10 : Détection des anticorps dressés contre la protéine F du virus HIV dans les sérums de 2 patients suivis au cours du temps (3 prélèvements).

Figure 11A : Distribution isotypique des anticorps reconnaissant l'antigène F et les peptides dérivés de F dans les serums de 6 individus à risque, séronégatifs.

Figure 11B : Distribution isotypique des anticorps reconnaissant les peptides dérivés de F dans les serums de 21 individus présentant des anticorps anti-F.

Exemple I Etude de la réponse isotypique d'individus contaminés par le virus HIV.

L'étude porte sur plus de 100 échantillons de sérums reconnus comme positifs dans les tests de diagnostic rapide par la technique d'ELISA (tests commercialisés par Abbott, Organon, Diagnostics Pasteur, Welcome), tests couramment utilisés dans les centres de transfusion sanguine.

La spécificité antigénique de la réponse (c'est-à-dire contre les protéines ENV, GAG ou POL) est analysée par "LAV-BLOT" (kit commercialisé par Diagnostics Pasteur).

Le test repose sur le principe de l'ELISA indirect sur nitrocellulose sensibilisée avec des protéines virales. Les protéines du virus LAV inactivé sont séparées par électrophorèse sur gel de polyacrylamide en milieu dissociant et réducteur puis transférées sur membrane de nitrocellulose. La mise en oeuvre du test comprend les étapes suivantes :

- Des bandelettes de nitrocellulose prédécoupées sont saturées à l'aide de lait écrémé pour éviter les adsorptions non spécifiques.

- Chaque échantillon à tester ou sérum de contrôle est mis à incuber avec une bandelette de nitrocellulose. Si des anticorps anti LAV sont présents, ils se lient aux antigènes viraux fixés sur la bandelette.

- Après lavage, un anticorps anti-IgG humaines, marqué à la peroxydase, est ajouté et mis à incuber. Il se lie aux anticorps anti LAV retenus sur le support solide.

- L'activité des complexes liés à la nitrocellulose est révélée, après lavage, par addition du substrat de l'enzyme.

- La présence d'anticorps anti LAV dans le sérum se traduit par l'apparition de bandes colorées spécifiques. La position des bandes colorées correspond aux masses moléculaires des protéines virales fixées sur la nitrocellulose. Les protéines reconnues par le test sont : GP160, précurseur de la GP110 ; GP110, enveloppe du virus ; P68, polymérase ; P55, précurseur des protéines internes (GAG) ; GP41, protéine transmembranaire ; P40, précurseur des protéines internes ; P34, endonucléase ; P25, P18 et P13 protéines internes.

- L'interprétation des résultats se fait en alignant les bandelettes du test avec celle du témoin séropositif.

Le test LAV-BLOT révèle les anticorps de classe IgG. Si des anticorps d'un autre sous-type sont fixés sur les bandelettes de nitrocellulose, ils ne sont pas détectés.

Le test peut donc être amélioré par la détection des IgA, IgM et IgE ; de plus au sein des anticorps de classe IgG on peut raffiner la détection en révélant spécifiquement les isotypes IgG1, IgG3 ou IgG4.

La base technique de l'étude repose sur l'utilisation en test "Western Blot" d'anticorps monoclonaux dirigés contre les classes ou les sous-classes d'anticorps humains, à l'exception des IgG2 dont l'anticorps monoclonal est reconnu comme dépourvu d'activité.

Pour chaque sérum qui justifie une analyse détaillée, il convient donc de réaliser 6 tests en parallèle sur les bandelettes de nitrocellulose du kit LAV-BLOT et ensuite de révéler les anticorps fixés à l'aide d'anti-anticorps spécifiques. Chaque bandelette est révélée avec un anti-anticorps. Les anti-IgA sont vendus par TAGO-BIOSOFT ; les anti-IgM sont vendus par MILES ; les anti-IgG1, IgG3 et IgG4 sont vendus par Seward.

- La première étape d'adsorption du sérum des patients sur les bandelettes est réalisée conformément aux instructions du fournisseur (LAV-BLOT) : après saturation de la nitrocellulose en présence de PBS + lait 5 %, pH 7,4, les sérums dilués au 1/50ème sont incubés sous agitation continue, une heure à température ambiante puis une nuit à 4°C. Les bandelettes sont ensuite lavées 3 fois pendant 10 minu tes. On ajoute ensuite l'anticorps spécifique de chaque classe ou sous-classe.

- Détection des IgM et des IgA :
l'anticorps monoclonal anti-IgA (vendu par TAGO-BIOSOFT), spécifique de Fc , est utilisé à la dilution 1/1000 ; l'anticorps monoclonal anti-IgM (vendu par MILES), spécifique de Fc , est utilisé à la dilution 1/1000 ; ces anticorps sont marqués à la peroxydase ; après incubation à température ambiante pendant 3 heures, les bandelettes sont lavées 3 fois puis l'activité peroxydase est révélée par une réaction colorée avec 60 mg HRP (HRP-color : 4 chloro-1-naphtol, fourni par BIO-RAD) + 20 ml éthanol + 100 ml PBS.

- Détection des IgG de sous-classe 1, 3 ou 4 :
les anticorps monoclonaux anti-IgG1, anti-IgG3, anti-IgG4 (vendus par Seward) sont utilisés à la dilution 1/500. Ces anticorps ne sont pas marqués.

Aprés 3 heures d'incubation, les anticorps fixés sont révélés par un anticorps "anti-souris" marqué à la peroxydase (anticorps fourni par Diagnostics Pasteur). Aprés 3 lavages, l'activité peroxydase est révélée, comme dans le test précédent.

- Détection des IgE :
la première étape d'adsorption du sérum des patients est prolongée pendant 5 heures (au lieu d'une) ; l'anticorps monoclonal anti-IgE (vendu par TAGO-BIOSOFT) est utilisé à la dilution 1/250. Cet anticorps est marqué à la peroxydase. Il est révélé comme décrit plus haut.

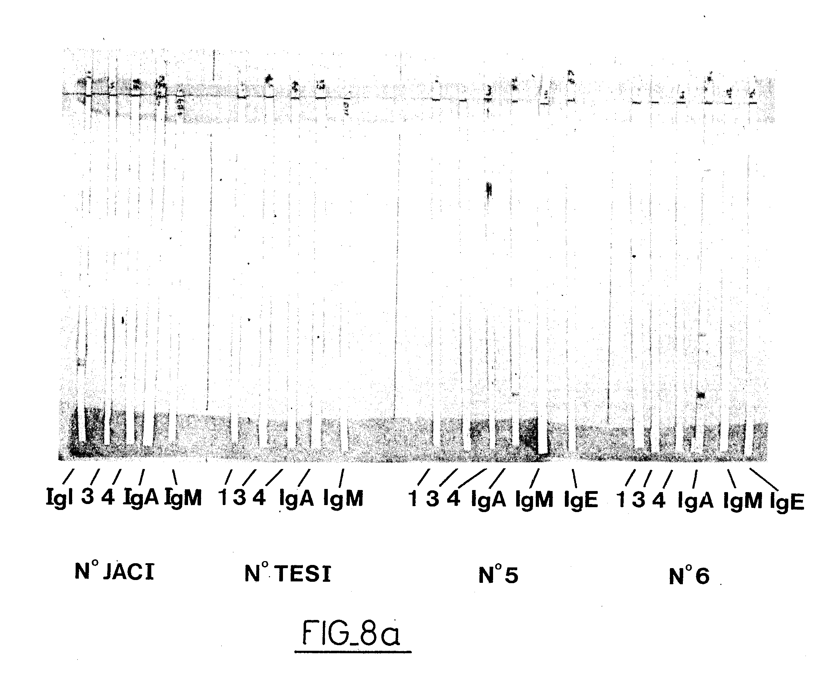

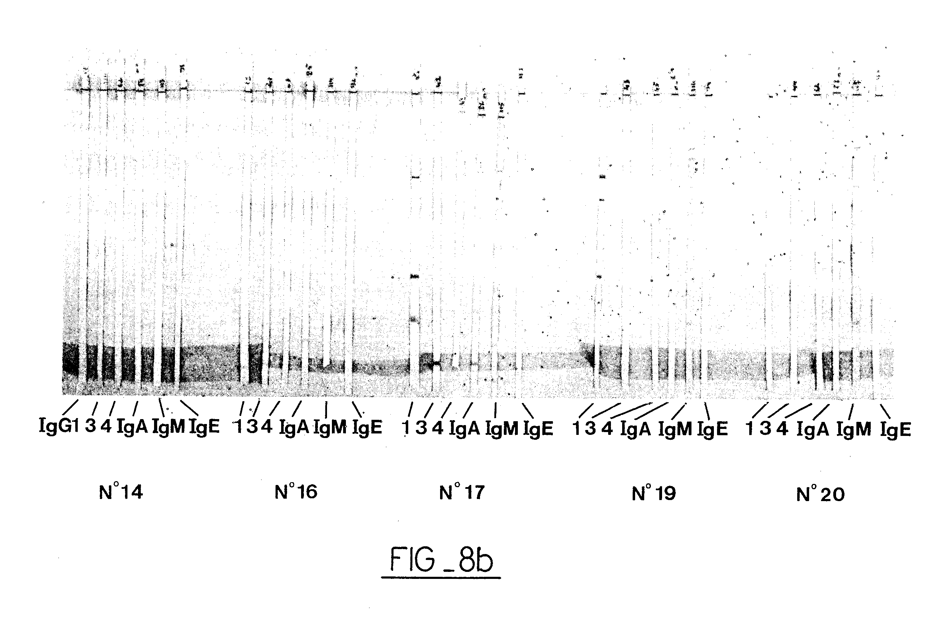

Après réaction de la peroxydase, les bandelettes sont alignées avec le témoin positif du kit LAV-BLOT pour localiser quels antigènes ont fixé les anticorps spécifiques. La figure 8 illustre cette expérience. Les résultats sont discutés dans l'exemple 3.

Exemple 2 Mise au point d'un test d'immunodétection de l'antigène F du virus HIV.

L'antigène F du virus HIV ne fait pas partie des protéines qui sont fixées sur les bandelettes de nitrocellulose du kit LAV-BLOT.

Un test semblable peut être préparé avec de l'antigène F purifié, préparé après manipulation génétique et expression dans E. coli.

La séquence codante de la protéine F a été insérée dans un vecteur M13 : M13TG170 (brevet français 86.08698). Un site BamHI a été créé en amont de l'ATG d'initiation, par mutagénèse localisée avec l'oligonucléotide suivant :

5′ GCCACCCATAGGATCCCAAATCCTTT 3′

La construction portant la mutation est appelée M13TG1106. On récupère la séquence codante de la protéine F sous forme d'un fragment BamHI-HindIII et on l'introduit dans un plasmide construit pour permettre l'expression d'un gène étranger dans E. coli, sous la dépendance du promoteur thermoinductible $P_L$ du phage λ. On utilise par exemple le plasmide pTG959 mais un autre vecteur pourrait aussi être utilisé. (Le plasmide pTG959 dérive des plasmides pTG908 et pTG956 décrits dans le brevet français 83.00909 ou PCT/FR84/00014 ; la séquence rbs-cII du pTG908 a été remplacée par la séquence rbs synthétique du pTG956, après délétion d'un fragment HpaI-NdeI qui raccourcit le gène N. Les éléments utiles de cette construction sont les suivants :

P$_L$-N'-rbs synthétique-ATG-CII'-polylinker ...

↑
site BamHI

La séquence codante à exprimer est introduite dans le polylinker).

Le plasmide résultant est appelé pTG1166.

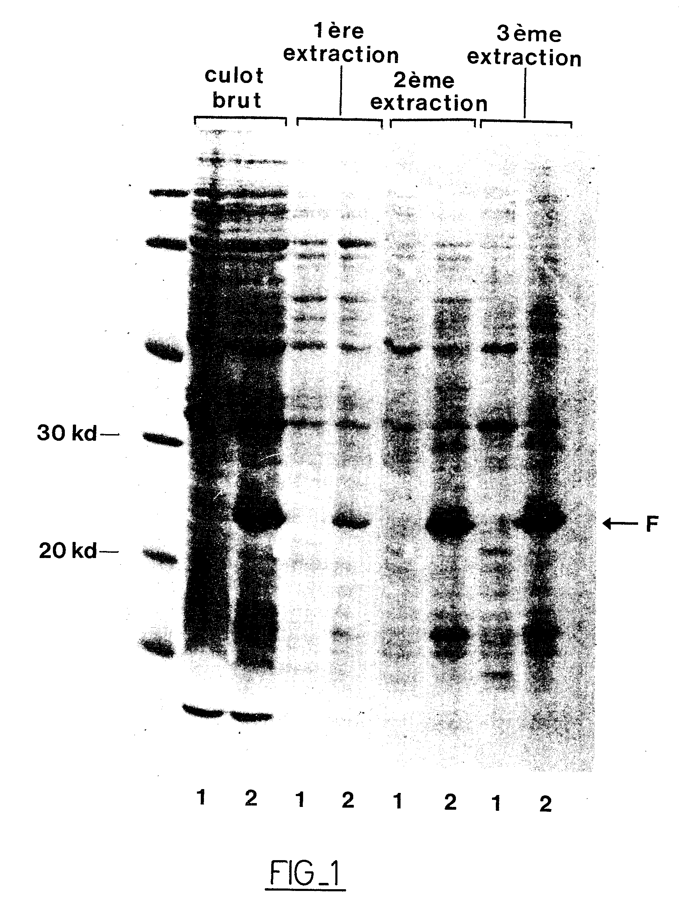

Après transformation de la souche E. coli TGE901, l'induction de la culture à 42°C assure une expression de la protéine F à un taux de 20 % des protéines totales (voir figure 1).

La protéine F est récupérée après centrifugation dé la culture (10 minutes à 6.000 rpm) et sonication du culot repris dans 10 ml de PBS (pour 500 ml de culture de départ) pendant 8 minutes en continu. Le culot est ensuite repris dans 2 ml de PBS + 0,2 % SDS. Après 10 minutes d'incubation à 25°C, la préparation est centrifugée (10 minutes à 10.000 rpm) et le surnageant est récupéré. L'opération est renouvelée deux fois successivement sur le culot restant.

Les surnageants des 3 extractions sont soumis à l'électrophorèse sur gel d'acrylamide 15 % - SDS. On voit (Figure 1) que c'est par la seconde extraction qu'on obtient la protéine la plus pure (à environ 70 %), à une concentration d'environ 1 µg/µl.

La protéine ainsi purifiée est transférée du gel sur nitrocellulose. La nitrocellulose est ensuite découpée en bandelettes pour réaliser les tests d'immunodétection. On a donc réalisé un test d'immunodétection semblable au LAV-BLOT mais spécifique de la protéine F.

Les bandelettes sont ensuite incubées avec les serums de patients à étudier, suivant le même protocole que pour le LAV-BLOT.

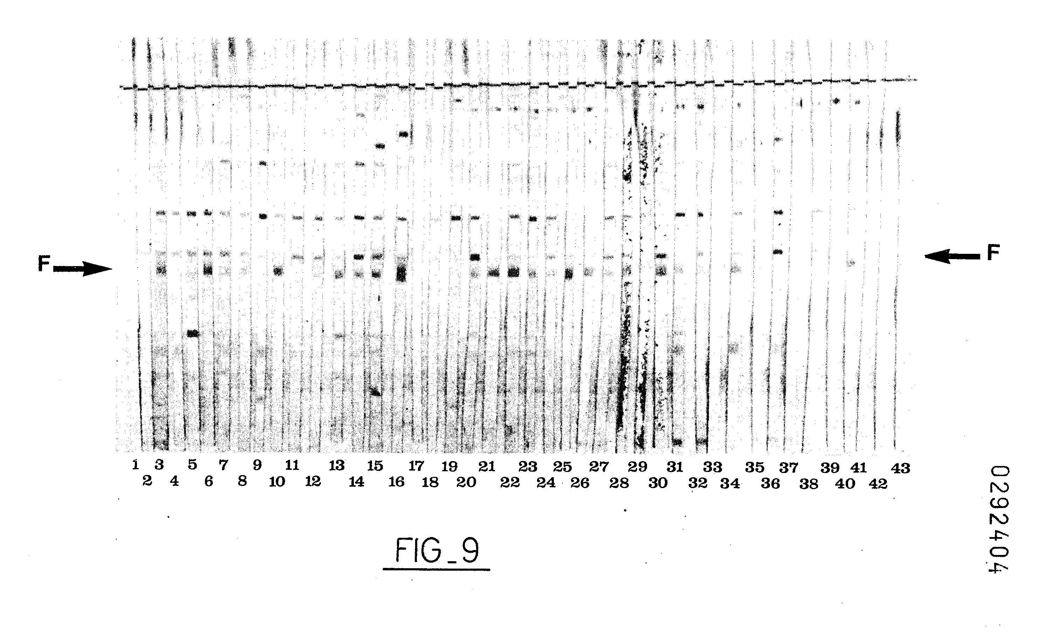

Les anticorps fixés peuvent ensuite être révélés, comme dans le LAV-BLOT, avec des anti-IgG totales, marqués à la peroxydase (Figure 9).

L'immunodétection peut aussi être réalisée sur plusieurs bandelettes en parallèle, pour chaque sérum, pour révéler les anticorps de différents isotypes, comme décrit dans l'exemple 1.

L'antigène F peut également être remplacé par des peptides dérivés de celui-ci et qui correspondent à des épitopes importants. Ces peptides peuvent être synthétisés in vitro, selon des méthodes classiques.

On a choisi en particulier 2 peptides correspondant aux acides aminés 45 à 69 et 98 à 112 de la séquence de l'antigène F. La séquence de ces peptides est la suivante :

45 à 69 : SSNTAATNAACAWLEAQEEEEVGFP

98 à 112 : EGLIHSQRRQDILDL

L'avantage des peptides synthétisés in vitro réside dans l'absence totale de contaminants d'origine bactérienne et donc dans leur grande spécificité.

Exemple 3 Analyse de la réponse isotypique contre les différentes protéines du HIV dans les serums de patients contaminés ou "à haut risque".

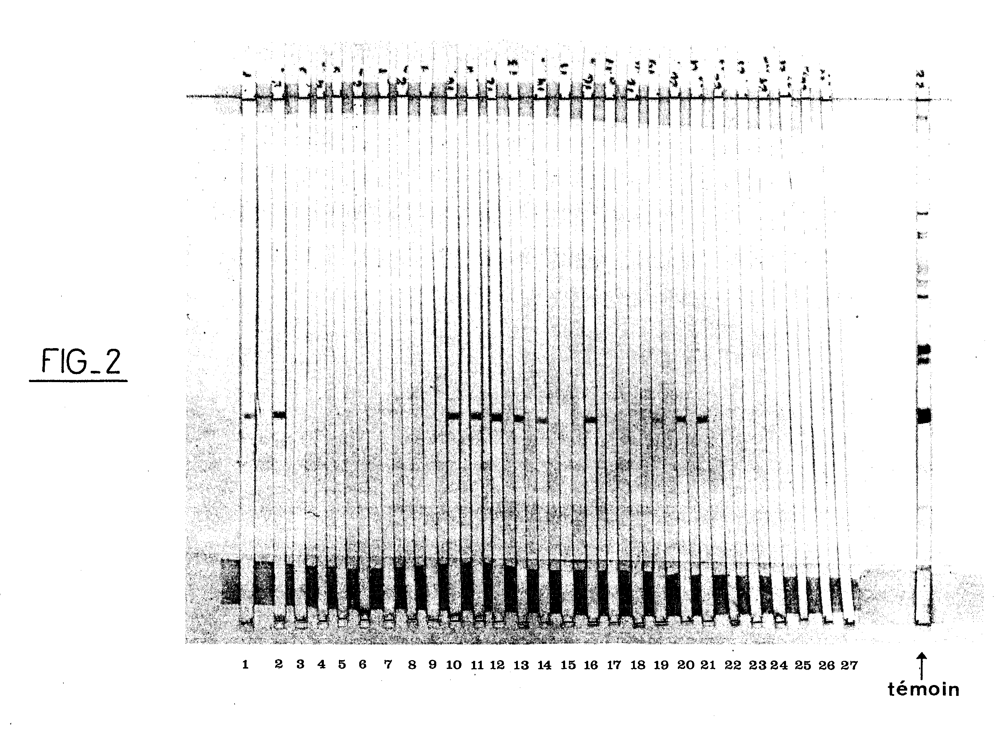

Dans une étude portant sur une série de serums de patients contaminés, (de 22 à 111 sérums selon les tests - voir figures 2 à 8) la réponse de classe et de sous-classe ou réponse isotypique a été analysée, selon la méthodologie décrite dans l'exemple 1.

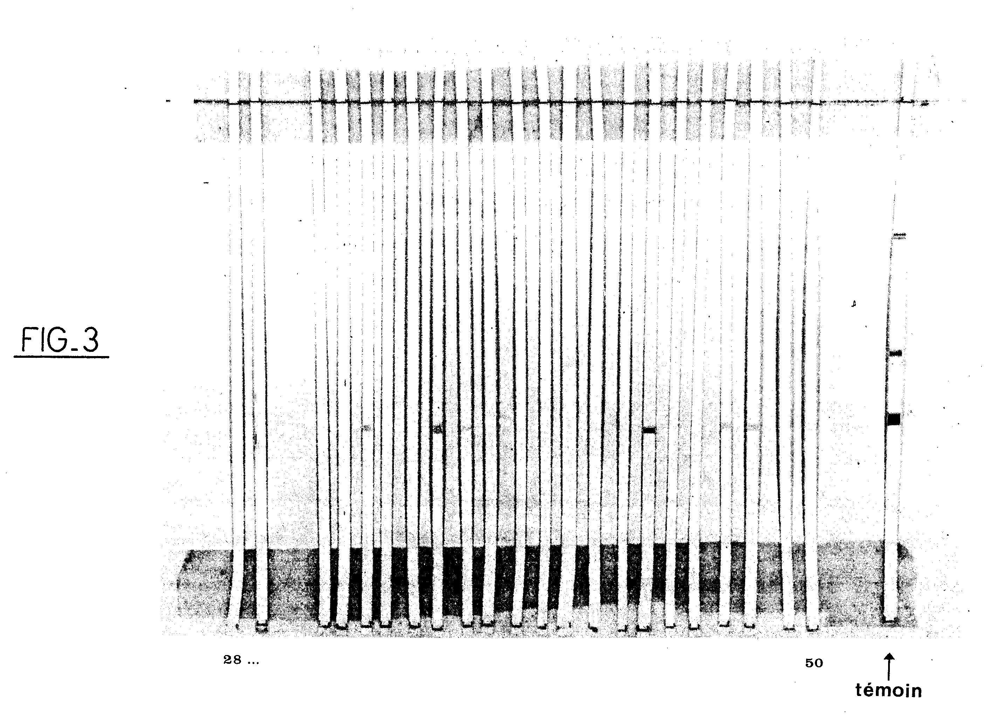

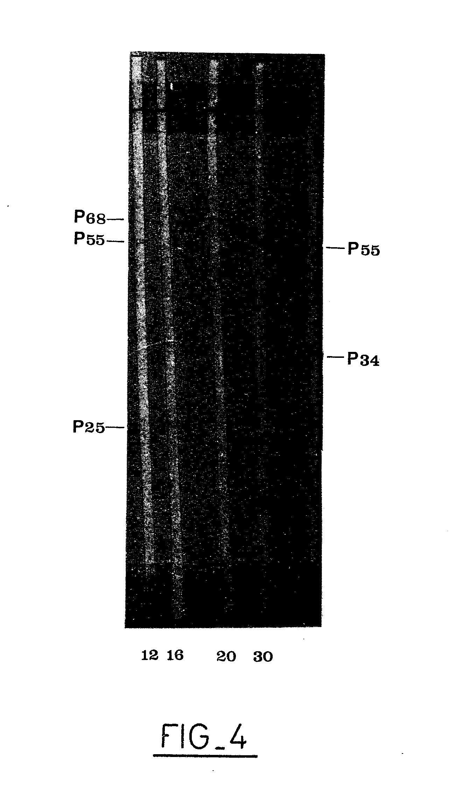

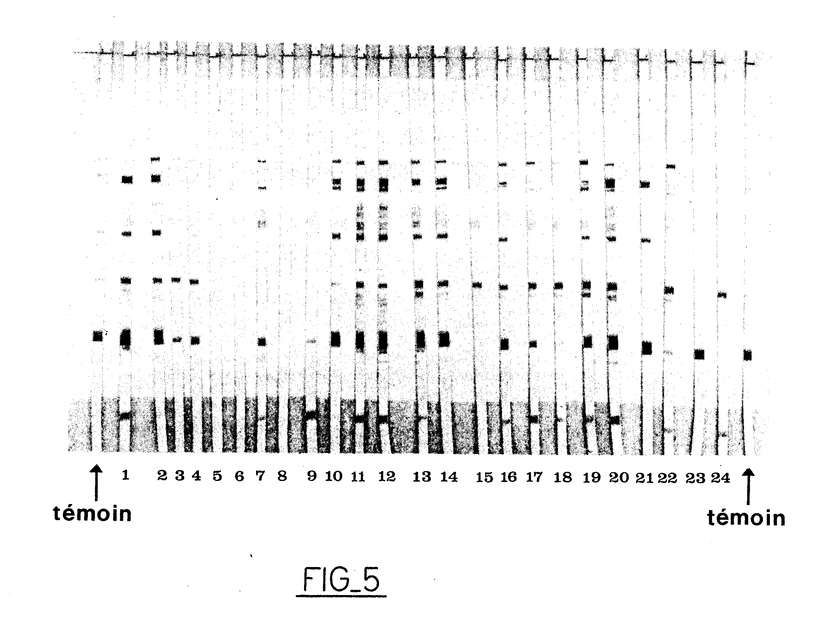

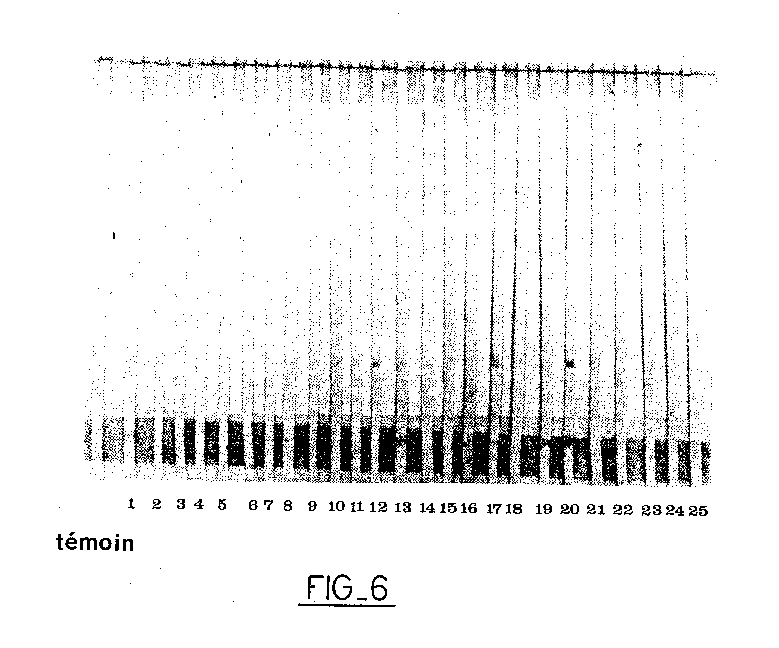

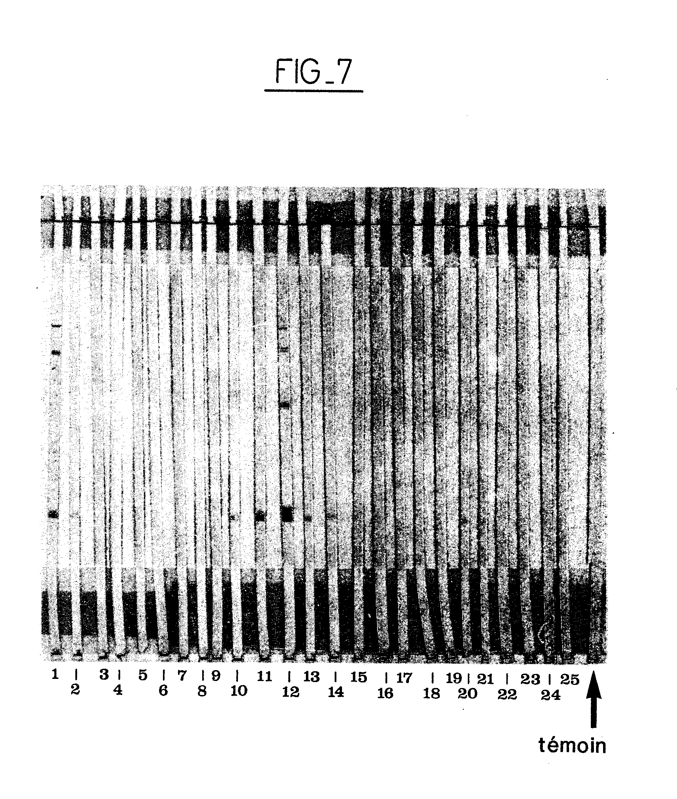

Les isotypes révélés sont les IgA (figure 2), les IgM (figure 3), les IgE (figure 4), les IgG1 (figure 5), les IgG3 (figure 6) et les IgG4 (figure 7).

Le tableau I résume les pourcentages de réponses positives observées pour les divers isotypes, contre les protéines ENV, GAG et POL, avec 22 serums qui donnent des réponses claires dans les figures précédentes.

Le tableau II résume les pourcentages de réponses positives observées en particulier pour les isotypes IgA, IgM et IgE, sur un plus grand nombre d'échantillons (111 ou 85).

Tableau I    Réponse en anticorps anti-HIV : % des serums positifs parmi 22 serums.

| Antigène | Isotype | | | | | |
|----------|---------|---------|---------|---------|---------|---------|
|          | IgG1    | IgG3    | IgG4    | IgA     | IgM     | IgE     |
| ENV      | 54 %    | 0 %     | 13 %    | 9 %     | 4 %     | 0 %     |
| GAG      | 54 %    | 45 %    | 0 %     | 63 %    | 36 %    | 8 %     |
| POL      | 40 %    | 0 %     | 18 %    | 45 %    | 36 %    | 6 %     |

Tableau II    Réponse en anticorps anti-HIV.
(nombre de sérums analysés : n=111 ou n=85)

| Antigène | Isotype | | | % total de serums positifs, tous isotypes cumulés |
|----------|---------|---------|---------|---------------------------------------------------|
|          | IgA     | IgM     | IgE     |                                                   |
| ENV      | 2 %     | 1 %     | 0 %     | 27 %                                              |
| GAG      | 44 %    | 33 %    | 8 %     | 76 %                                              |
| POL      | 12 %    | 9 %     | 6 %     | 44 %                                              |
| % total de sérums positifs | 46 %  n=111 | 34 %  n=111 | 11 %  n=85 |                                  |

L'analyse de ces résultats fait apparaître une restriction isotypique très nette.

1) La réponse dirigée contre les antigènes ENV est relativement restreinte à la sous-classe IgG1 ; on observe aussi une réponse IgG4 qui est parfois importante.

2) On observe une réponse postive dirigée contre les protéines GAG avec presque tous les isotypes, à l'exception des IgG4. Les réponses IgA et IgM sont très nettes (surtout contre la P25) ; les réponses IgE sont plus rares.

3) La réponse dirigée contre les protéines POL (P34 et P68) n'est jamais de type IgG3 ; tous les autres isotypes sont représentés.

Les protéines GAG et POL stimulent de manière importante les réponses IgA et IgM (et, en outre, les IgG). De plus, la production d'anticorps IgA ou IgM peut être observée en l'absence de reconnaissance par les IgG (Tableau II et figure 8 : patients N°6 et N°20).

La mise en évidence de réponse IgA ou IgM en absence de réponse IgG souligne une des limites du test de

0 292 404

diagnostic actuellement utilisé et dans lequel ces patients apparaissent faussement comme séronégatifs.

D'autre part l'importante réponse de type IgA, IgM et IgG4 de plusieurs serums permet d'envisager une étude affinée de la réponse immunitaire, au cours du temps. On sait en effet que les anticorps IgA, IgM et IgG4 sont souvent des anticorps bloquants (en particulier dans le cas des maladies parasitaires, comme décrit plus haut). De plus ces anticorps IgA et IgM sont dans 95 et 97 % des cas (Tableau II) dirigés contre les protéines GAG (surtout P25), c'est-à-dire les protéines internes, ce qui renforce l'analogie avec les maladies parasitaires (Il faut noter que la présence d'anticorps bloquants peut expliquer les faibles taux d'anticorps séroneutralisants détectés dans les sérums).

Une évolution de la réponse immunitiare vers ces isotypes bloquants pourrait donc être un marqueur de pronostic défavorable dans l'évolution de la maladie.

Exemple 4 Détection des anticorps dirigés contre la protéine F du virus HIV dans le sérum de patients contaminés ou "à haut risque".

La méthodologie décrite dans l'exemple 2 a été appliquée à l'analyse d'une série de sérums de patients.

Une série de 43 sérums a été analysée et révélée de manière globale avec des anti-IgG totales. Les résultats sont présentés dans la figure 9. La réponse dirigée contre la protéine F est bien visible ; le bruit de fond correspond à des réponses non spécifiques contre les protéines de E. coli qui sont fixées sur les bandelettes (la protéine F extraite de E. coli n'étant pure qu'à 70 %, comme décrit dans l'exemple 2).

Une étude plus précise a été effectuée sur un nombre restreint de sérums et montre également une restriction isotypique :

| Isotype | Réponse en anticorps anti F (parmi 11 sérums) |
|---------|-----------------------------------------------|
| IgG1 | 90 % |
| IgG3 | 0 % |
| IgG4 | 36 % |
| IgA | 63 % |
| IgM | (non significatif) |
| IgE | 28 % |

La réponse isotypique dressée contre F ressemble donc à la fois à la réponse contre les protéines de surface (ENV) et contre les protéines internes (GAG), puisqu'on observe une forte réponse IgG1 et IgG4 et IgA. Il n'y a aucune réponse IgG3 et la réponse IgM est faible ou absente.

L'analyse du sérum de quelques patients qui ont été suivis au cours du temps et depuis le début de leur séroconversion montre que les anticorps anti F apparaissent très précocément (voir exemple 5).

La détection de ces anticorps présente donc un intérêt majeur dans l'établissement du diagnostic du SIDA.

Exemple 5 Détection précoce d'anticorps contre la protéine F du virus HIV.

Une analyse préliminaire des sérums, de quelques patients considérés a priori comme "à haut risque" et qui ont été suivis au cours du temps montre que la séroconversion contre la protéine F se produit très précocément, en même temps ou même avant la séroconversion contre les protéines GAG.

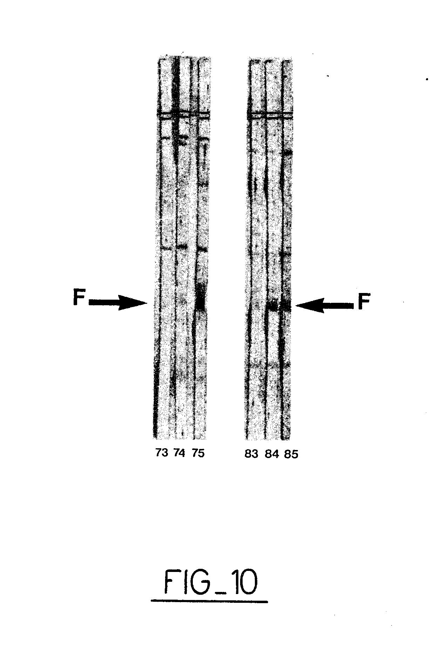

La figure 10 montre la détection des anticorps anti F (révélés avec des anti-IgG totales) dans 3 prélèvements de sérum successifs de 2 patients "à haut risque".

Le patient 1 (bandelettes 73, 74, 75 correspondant aux prélèvements du 4/12/1985, du 17/6/1986 et du 2/8/1986) est faiblement positif dès le deuxième prélèvement et fortement positif au troisième prélèvement. Les 3 mêmes sérums analysés par test de routine ELISA (au centre de transfusion sanguine) étaient négatif, faiblement positif et positif.

Le patient 2 (bandelettes 83, 84, 85 correspondant aux prélèvements du 5/1/1987, du 20/1/1987 et du 12/2/1987) est faiblement positif dès le premier prélèvement et nettement positif ensuite.

Les mêmes 3 sérums analysés par test ELISA étaient négatif, faiblement positif ou très douteux (ELISA fourni par Organon (±) Pasteur (±) et Welcome (-)) et positif ou douteux (Welcome) selon le test.

Les mêmes sérums avaient été confirmés en test "Western Blot" (fourni par Du Pont de Nemours) : le premier prélèvement était négatif pour tous les antigènes, le deuxième était douteux, légèrement positif pour P18 P25 P55 et P68, le troisième était positif.

Pour l'un de ces patients on a approfondi l'étude par la détection de l'ADN du virus HIV dans les cellules sanguines mononucléées et le test s'est révélé positif [≧ 1 cellule sur 100.000 positive par la technique de PCR - "polymerase chain reaction"].

Ce résultat souligne l'importance de la détection des anticorps dressés contre la protéine F : ils donnent une réponse claire et très précoce ce qui permet donc de poser un diagnostic précoce de séropositivité SIDA pour des individus porteurs du virus qui sont actuellement considérés comme séronégatifs dans les tests pratiqués couramment dans les centres de transfusion sanguine.

Une autre étude portant sur 6 sujets à haut risque séronégatifs selon les techniques classiques, a été effectuée par dosage radioimmunologique de la présence d'anticorps d'isotypes IgG1, IgG4, IgM, IgA et IgE dirigés contre la protéine F. Les 2 peptides 45-69 et 98-112 dérivés de F ont également été inclus dans le test. Cette étude confirme qu'il est possible de détecter une séroconversion précoce (et donc une contamination par le virus) chez des individus considérés comme sains.

Le dosage radioimmunologique se fait en utilisant la protéine F recombinante produite dans E. coli et purifiée à plus de 90 % ou un des peptides synthétiques, adsorbés sur une surface solide. Brièvement, 5 µg de peptide (ou de protéine recombinante F) sont incubés pendant 72 h dans 250 µl de carbonate 0-015M/Bicarbonate 0-035 M, à pH 9.6, dans des tubes à ailettes (Nunc). Toutes les incubations se font à température ambiante. Les tubes sont ensuite décantés, saturés par 250 µl de PBS-BSA à 3 %, pendant 2 heures, puis lavés. Le sérum à tester, dilué au 1/50e dans un volume final de 250 µl de PBS-BSA à 3 %, est introduit pendant une nuit, puis 2 lavages en PBS sont pratiqués. Enfin, un anticorps anti-isotype humain, radiomarqué à l'iode 125 (environ 100.000 coups pour 15 à 20 ng d'anticorps) est introduit pendant une nuit dans un volume final de 250 µl de PBS-BSA 3 %. Ces anticorps anti-isotype humain sont soit des anticorps polyclonaux de lapin, purifiés par affinité et dirigés contre l'isotype humain IgM, IgA ou IgE (respectivement spécifiques de la chaîne µ, α ou ε) (Miab, Upsala, Suède) ; soit des anticorps monoclonaux de souris dirigés contre la sous-classe humaine IgG1 ou IgG4 (Seward, U.K.). Deux lavages en PBS-Tween 0.1 % sont ensuite pratiqués et la radioactivité restant fixée dans le tube est comptée dans un compteur gamma (LKB).

Les résultats sont appréciés en pourcentage de radioactivité fixée. Une dizaine de sérums humains témoins, provenant de sujets sains séronégatifs pour les protéines structurales et non structurales (F et Sor) du HIV (testés en "Western Blot") sont étudiés simultanément. La limite supérieure de la normale (fixation non spécifique) est calculée comme la moyenne de la fixation obtenue avec les sérums témoins, plus deux fois l'écart type à la moyenne.

La détection, dans un but diagnostique, d'anticorps dirigés contre le peptide 45-69, pourra être adaptée à une technique plus simple de type ELISA ou Dot Blot.

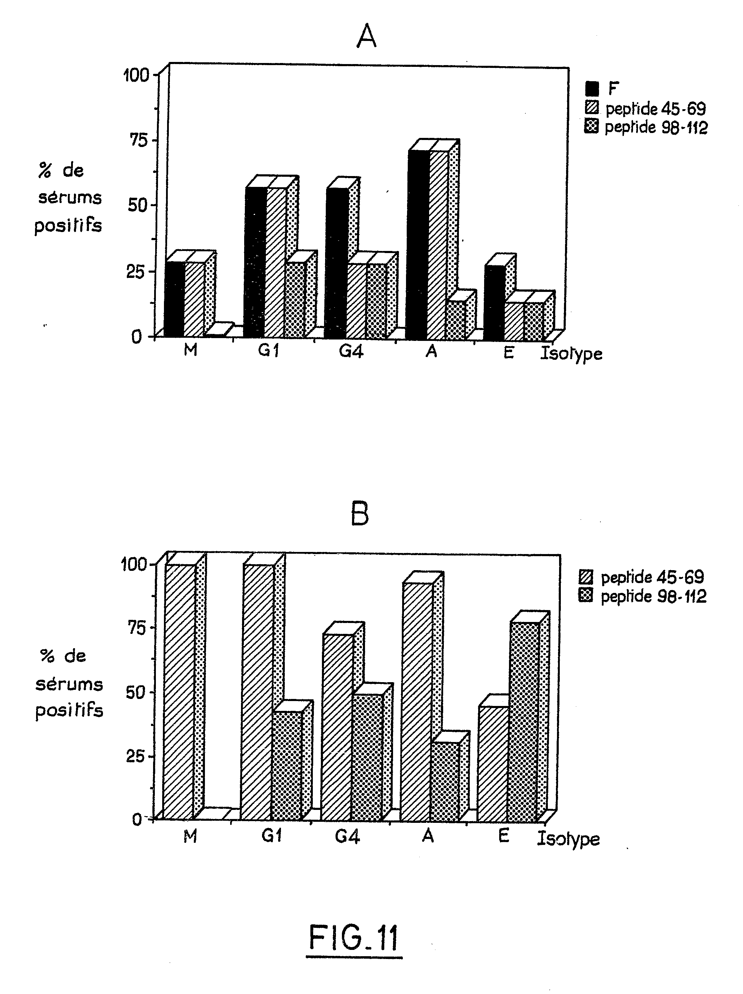

Le dosage radioimmunologique montre que les anticorps anti-F d'isotype IgM et IgA (réponse précoce) et IgG1 reconnaissent tous (IgM et IgG1) et presque tous (IgA) le peptide 45-69 de F. Ce peptide présente donc un intérêt diagnostique dans la mesure où il permet de détecter 3 isotypes majeurs des anticorps anti-F, aussi bien lorsque ces anticorps sont présents de manière isolée (n = 6), que chez des individus séropositifs par les techniques classiques (n = 8) (21 sérums étudiés au total, chez 14 individus) (Fig. 11A et 11B).

Le peptide 98-112 n'est reconnu par aucun anticorps anti-F d'isotype IgM, par une faible proportion des anticorps d'isotype IgA, une forte proportion des IgG4, et la majorité des IgE. Il est aussi reconnu par un anticorps monoclonal de rat, d'isotype IgG2a, appelé $FM_{11}$ obtenu à partir de rats immunisés par la protéine F recombinante.

Comme on l'a montré plus haut, les tests de diagnostic rapide (ELISA, fournis par Abbott, Organon, Diagnostics Pasteur, Welcome ...) ou de diagnostic précis par "Western Blot" (Diagnostics Pasteur, Du Pont de Nemours ...) peuvent donner de faux résultats séronégatifs.

D'autre part les kits de diagnostic actuellement commercialisés sont préparés avec des protéines purifiées à partir de virus HIV, inactivé ; ces tests coûtent donc très cher.

Il apparaît, dans l'analyse de la spécificité antigénique de la réponse anticorps que tous les sérums positifs donnent une réponse contre les protéines GAG (hormis dans les stades terminaux de la maladie).

Il serait donc possible de remplacer l'ensemble des protéines virales adsorbées dans la nitrocellulose par les protéines GAG, en particulier la P25, et ENV. Comme ces 2 protéines ont déjà été exprimées par manipulation génétique (brevets français 86.05043 et 86.16787), il est possible d'utiliser ces protéines purifiées, dans des conditions beaucoup plus économiques et moins dangereuses que lors de la purification à partir du virus. - Un test de diagnostic simple, clair et précoce peut comprendre les protéines GAG, Env et F (préparées in vitro par manipulation génétique).

- Un test d'analyse détaillée, permettant l'étude évolutive au cours de la maladie, de la réponse isotypique, devrait comprendre

. l'immunocapture des anticorps dressés contre toutes les protéines du virus HIV y compris la protéine F ou des peptides dérivés de celle-ci

. et la détection des anticorps fixés à l'aide des anti-anticorps spécifiques de tous les isotypes.

L'analyse au cours du temps de la réponse isotypique pourrait aussi aboutir à une nouvelle stratégie vaccinale.

REFERENCES

Cebra, J.J., Komisar, J.L. and Schweitzer, P.A. Ann. Rev. Immunol. 2, 493-548 (1984).

Capron, M. and Capron, A. Parasitology Today 2, 69 (1986).

Grzych, J.M., Capron, M., Dissous, C. and Capron, A. J. Immunol. 133, 998 (1984).

Khalife, J., Capron, M., Capron, A., Grzych, J.M., Butterworth, A.E., Dunne, D.W. and Ouma, J.H. J. Exp. Med. 164, 1626-1640 (1986).

Capron, M., 1983, et Haque, A., Capron, A., Ouaissi, A., Kouememi, L., Lejeune, J.P., Bonnel, B. et Pierce, R., 1983, dans le compte rendu du Colloque d'Antwerp : "From parasitic infection to parasitic disease" - Contributions to microbiology and immunology vol. 7, Ed. Gigasse et Van Marck p. 1 et 2 et p. 9 à 21.

**Revendications**

1) Procédé de diagnostic in vitro du SIDA chez un patient, par mise en évidence des anticorps dressés contre certaines protéines spécifiques du virus HIV dans un prélèvement biologique dudit patient, selon un processus d'immunodétection indirect dans lequel :

a) on fixe la ou lesdites protéines du virus HIV, préalablement isolées, sur un support,

b) on met en présence ces protéines fixées avec le prélèvement biologique dudit patient pouvant contenir lesdits anticorps, et

c) après l'immunocapture des anticorps, on détecte la présence des anticorps fixés à l'aide d'anti-anticorps marqués,

caractérisé en ce que parmi les anti-anticorps marqués assurant la détection l'un au moins est spécifique d'un isotype.

2) Procédé selon la revendication 1, caractérisé en ce qu'on utilise au moins deux anti-anticorps marqués spécifiques chacun d'un isotype différent, les anti-anticorps pouvant être utilisés séparément ou en combinaison pour la détection.

3) Procédé selon l'une des revendications 1 et 2, caractérisé en ce que les anti-anticorps sont marqués par une enzyme et en ce que la détection est effectuée par addition du substrat de l'enzyme, ou que les anti-anticorps sont marqués par un isotope radioactif.

4) Procédé selon l'une des revendications 1 à 3, caractérisé en ce que parmi les isotypes détectés figure au moins l'un des isotypes IgA, IgM ou IgE.

5) Procédé selon l'une des revendications 1 à 4, caractérisé en ce que parmi les isotypes détectés figure au moins l'un des isotypes IgG1, IgG3 ou IgG4.

6) Procédé selon l'une des revendications 1 à 5, caractérisé en ce que parmi les protéines fixées une ou plusieurs des protéines correspondant au gène GAG, POL, ENV ou les peptides dérivés de ces protéines.

7) Procédé selon la revendication 6, caractérisé en ce que parmi les protéines fixées figure la protéine F ou des peptides dérivés de cette protéine.

8) Procédé selon les revendications 6 et 7, caractérisé en ce que les protéines fixées sont obtenues par recombinaison génétique.

9) Peptides dérivés de la protéine F caractérisés en ce qu'ils sont choisis parmi les peptides de séquence :

SSNTAATNAACAWLEAQEEEEVGFP

EGLIHSQRRQDILDL

10) Procédé selon la revendication 7 caractérisé en ce que parmi les peptides dérivés de la protéine F on choisit les peptides selon la revendication 9.

11) Procédé selon l'une des revendications 1 à 8, caractérisé en ce que les protéines fixées sont choisies parmi les protéines correspondant au gène GAG, ENV et la protéine p25, et en ce que les isotypes sont choisis parmi IgG1, IgG3, IgG4, gA, IgM et IgE.

12) Procédé selon l'une des revendications 1 à 10, caractérisé en ce que la protéine fixée est choisie parmi la protéine env ou la protéine F et en ce que les isotypes sont choisis parmi IgG1, IgG4, IgA et IgE.

13) Trousse de diagnostic in vitro du SIDA pour la mise en oeuvre du procédé selon l'une des revendications 1 à 11, caractérisée en ce qu'elle comprend au moins une ou plusieurs protéines du virus HIV préalablement isolées choisies parmi les protéines correspondant au gène :

- GAG (p55, p40, p25, p18 et p13)

- POL

- ENV (gp160, gp120 et gp40)

- protéine F,

et au moins un anti-anticorps spécifique d'un isotype choisi parmi IgG1, IgG3, IgG4, IgA, IgM, IgE.

14) Trousse de diagnostic selon la revendication 13, caractérisée en ce qu'elle comporte la protéine F et la protéine env et au moins un anti-anticorps spécifique de l'isotype IgG1, IgG4, IgA ou IgE.

15) Trousse de diagnostic selon la revendication 4 caractérisée en ce que à la place de la protéine F, elle comporte des peptides dérivés de la protéine F.

16) Trousse de diagnostic selon la revendication 15 caractérisée en ce qu'elle comporte les peptides selon la revendication 9.

17) Trousse de diagnostic selon la revendication 13, caractérisée en ce qu'elle comporte au moins la protéine env et la protéine p25 ou la protéine correspondant au gène GAG et au moins un anti-anticorps spécifique de l'isotype IgG1, IgG3, IgG4, IgA, IgE et/ou IgM.

18) Trousse de diagnostic selon l'une des revendications 13 à 17, caractérisée en ce qu'il s'agit d'une trousse de diagnostic Western Blot ou ELISA.

19) Vaccin destiné au traitement et à la prévention des affections liées à la présence d'un virus HIV chez un patient, caractérisé en ce qu'on utilise comme agent vaccinant une protéine antigénique du virus HIV modifiée pour induire des anticorps d'isotypes effecteurs et non bloquants.

20) Vaccin selon la revendication 19, caractérisé en ce que la protéine antigénique du virus HIV est modifiée pour induire des anticorps d'isotypes IgG1, IgG2, IgG3 ou IgE.

21) Vaccin selon la revendication 20, caractérisé en ce que la protéine antigénique du virus HIV modifiée est la protéine correspondant au gène GAG, POL, ENV, F ou Q ou les peptides dérivés de ces protéines.

22) Vaccin selon l'une des revendications 19 à 21, caractérisé en ce que la protéine du virus HIV est modifiée pour induire des anticorps d'isotypes non bloquants.

23) Anticorps destinés au traitement des individus séropositifs, caractérisés en ce qu'ils sont effecteurs et non bloquants et dirigés contre une ou plusieurs protéines du HIV.

24) Anticorps selon la revendication 23 destinés au traitement préventif des individus à risque.

25) Anticorps selon l'une des revendications 23 et 24, caractérisés en ce qu'ils sont induits par des protéines recombinantes modifiés selon l'une des revendications 16 à 19.

26) Anticorps selon l'une des revendications 23 et 24, caractérisés en ce qu'ils sont purifiés par élimination des isotypes bloquants à partir d'un serum contenant un mélange d'isotypes.

27) Anticorps destinés au traitement et à la prévention du SIDA, caractérisés en ce qu'ils sont inhibiteurs des anticorps bloquants.

28) Anticorps selon la revendication 27, caractérisés en ce qu'ils sont induits, chez l'animal, par des anticorps bloquants ou les épitopes correspondants.

29) Anticorps selon l'une des revendications 23 à 28, caractérisés en ce qu'ils peuvent être des anticorps monoclonaux.

0292404

FIG_1

FIG_2

1  2  3  4  5  6  7  8  9  10  11  12  13  14  15  16  17  18  19  20  21  22  23 24  25  26 27

témoin

0292404

FIG. 3

28 ...                                    50

↑
témoin

0292404

FIG_4

0292404

0292404

FIG_5

1107 88

0292404

FIG_6

0292404

# FIG_7

1 | 3 | 5 | 7 |9 | 11 | 13 | 15 | 17 | 19| 21 | 23 | 25
  2   4   6   8   10   12   14   16   18  20   22   24

témoin

Igl 3 4 IgA IgM    1 3 4 IgA IgM    1 3 4 IgA IgM IgE    1 3 4 IgA IgM IgE

N° JACI          N° TESI          N° 5          N° 6

FIG_8a

IgG1 3 4 IgA IgM IgE    1 3 4 IgA IgM IgE    1 3 4 IgA IgM IgE    1 3 4 IgA IgM IgE    1 3 4 IgA IgM IgE

N°14      N°16      N°17      N°19      N°20

## FIG _ 8b

FIG_9

0292404

FIG_10

0292404

## A

## B

## FIG.11

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | WO-A-8 604 993 (UNITED STATES OF AMERICA DEPARTMENT OF COMMERCE) * Page 3, lignes 2-18; page 4, lignes 10-23; page 5, lignes 10-14; page 8, lignes 2-34 * --- | 1,3,4,9 ,11-13, 20 | G 01 N 33/569 C 12 N 15/00 A 61 K 39/21 |
| X | EP-A-0 193 284 (UNITED STATES OF AMERICA DEPARTMENT OF COMMERCE) * Page 4, lignes 4-25; page 7, lignes 23-37; page 12, exemple 1; page 14, lignes 2-34 * --- | 3,5,23, 24,27- 29 | |
| X | EP-A-0 216 191 (ABBOTT LABORATORIES) * Colonne 2, ligne 27 - colonne 3, ligne 3; colonne 5, ligne 44 - colonne 6, ligne 51 * --- | 1,3,18 | |
| X | US-A-4 520 113 (R.C. GALLO et al.) * Colonne 2, ligne 58 - colonne 3, ligne 23; colonne 4, exemple 1; colonne 5, exemple 2; colonne 7, lignes 19-30,34-35; colonne 8, lignes 3-5 * --- | 1,3,9, 11,18 | |
| X | EP-A-0 199 301 (HOFFMANN-LA ROCHE & CO.) * Colonne 4, ligne 40 - colonne 5, ligne 22; colonne 16, ligne 36 - colonne 17, ligne 56; colonne 23, exemple 6 * --- | 1,6,8, 10,12, 19,21, 25 | G 01 N C 12 N A 61 K |
| X | EUROPEAN JOURNAL OF CLINICAL INVESTIGATION, vol. 17, no. 2, partie 2, 1987, page A40, Kiel, DE; G. HOBUSCH et al.: "Contribution of IgG-subclasses in healthy individuals pre-aids and aids" * Résumé * --- -/- | 1,2,5,9 -14,17, 20 | |

**DOMAINES TECHNIQUES RECHERCHES (Int. Cl.4)**

Le présent rapport a été établi pour toutes les revendications

| Lieu de la recherche | Date d'achèvement de la recherche | Examinateur |
|---|---|---|
| LA HAYE | 05-08-1988 | VAN BOHEMEN C.G. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X,P | WO-A-8 707 642 (TRANSGENE S.A. & INSTITUT PASTEUR) * Page 4, lignes 5-23; page 31, lignes 3-7,18-23; page 32, lignes 18-25 * | 1,7,10-12,14-16,21 | |
| X | EP-A-0 138 667 (INSTITUT PASTEUR) * Page 25, lignes 1-18 * | 1,6,9, 11,13, 17,18, 21,25 | |
| | | | **DOMAINES TECHNIQUES RECHERCHES (Int. Cl.4)** |

Le présent rapport a été établi pour toutes les revendications

| Lieu de la recherche | Date d'achèvement de la recherche | Examinateur |
|---|---|---|
| LA HAYE | 05-08-1988 | VAN BOHEMEN C.G. |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons
 
& : membre de la même famille, document correspondant

EPO FORM 1503 03.82 (P0402)